# EUROPEAN PATENT SPECIFICATION

(11) **EP 4 480 381 B1**
(45) Date of publication and mention of the grant of the patent: **23.09.2026**
(21) Application number: 23181218.1
(22) Date of filing: 23.06.2023
(51) Int. Cl.: A61B 1/00, A61B 1/015

(54) **ENDOSCOPE COMPRISING A FLUID PATHWAY SYSTEM**
ENDOSKOP MIT EINEM FLUIDPFADSYSTEM
ENDOSCOPE COMPRENANT UN SYSTÈME DE PASSAGE DE FLUIDE

(43) Date of publication of application: 25.12.2024
(73) Proprietor: Ambu A/S, 2750 Ballerup (DK)
(72) Inventor: MORTON, Alistair David, 2300 København (DK)
(74) Representative: Winter, Brandl - Partnerschaft mbB

(56) References cited:
- WO-A1-2014/089028
- WO-A1-2021/176675
- JP-A- 2006 026 248
- JP-A- H05 317 244
- JP-B2- 2 867 793
- US-A- 6 053 861
- US-A1- 2016 121 095
- US-A1- 2022 386 855
- US-A1- 2022 409 023
- US-B2- 10 052 472
- US-B2- 11 712 322

## Description

### TECHNICAL FIELD

The present invention relates to endoscopes, and in particular endoscopes comprising a fluid pathway system.

### BACKGROUND

Endoscopes are known and used for visual navigation into, and examination and diagnosis of, hollow organs and body cavities, as well as, optionally, to assist in surgery, e.g. for targeted tissue sampling. Endoscopes include procedure-specialized endoscopes, such as gastroscopes and bronchoscopes. Endoscopes may comprise a handle at the proximal end to be gripped by an operator and a flexible, elongated insertion cord extending distally from the handle. The insertion cord may include an insertion tube terminated in a distal tip part at the end of a highly bendable, e.g. articulated, bending section controllable by the operator. The tip part may comprise an observation optical system extending distally from the bending section. Some endoscopes include a fluid pathway for transporting media into or out of the body, e.g. delivery of a medical fluid or aspiration to remove fluids from a target position inside the body. The fluid pathway generally runs inside the endoscope from a position near the proximal end of the endoscope, i.e. outside the body, to a position near the distal end of the endoscope, i.e. inside the body.

Reusable endoscopes need to be thoroughly cleaned between each patient, and the fluid pathways are notoriously difficult to clean and hence the cleaning process is time-consuming. Over time biofilm may form in the fluid pathways, which may make the cleaning process virtually impossible and constitute a potential health hazard.

Single-use endoscopes, on the other hand, do not need to be sterilized after use. For single-use endoscopes, it is important that the entire device is manufactured in a cost-efficient way. Single-use endoscopes in the market are very capable and are widely used, but even such endoscopes can be further improved.

WO 2023/052237 A1 discloses an endoscope comprising a suction tube connected to Y-connector and valve and secured in position by adhesive. Connection by adhesive has widespread use, but there are some disadvantages to it, such as in relation to time consumption in manufacturing.

US 2022/409023 A1 discloses a connection joint for connecting tubes in an endoscope, which includes a sleeve for fitting to the tube, a pusher to apply bias to the sleeve, and a bias releasing portion to release the bias. The connection joint simplifies the connection and removal of tubes in an endoscope.

JP H05 317244 A discloses an endoscope channel cleaning device and an endoscope that prevent the pressure of the cleaning liquid in the liquid supply path or inside the endoscope channel from becoming too high during cleaning. This is achieved by providing a communication passage to release the pressure of the cleaning liquid.

US 2016/121095 A1 discloses a device for coupling a fluid source to a medical device, including a housing with proximal and distal portions and a deformable member on the distal portion to removably couple the housing to the medical device. The deformable member can create a fluid-tight seal, and the device aims to provide a reliable, long-lasting coupler that minimizes wear and tear.

US 2022/386855 A1 discloses an endoscope tubular connector that includes a connection joint with a through-hole and a softer tubular portion with one end arranged in the connection joint's through-hole, where the through-holes are connected and the tubular portion's outer circumference is within the connection joint's inner circumference.

Further, the endoscope should live up to high standards on the general performance of the endoscope, and it is desirable to improve versatility of endoscopes as well as reducing cost, particularly in single-use endoscopes, to increase their value.

### BRIEF DESCRIPTION OF THE DISCLOSURE

The objective of the present disclosure is to provide an endoscope with features that eliminate or at least reduce the disadvantages of the prior art and suitably deal with the problems mentioned above. In particular, an endoscope shall be provided, which is designed for single-use, and which has a fluid pathway system made of commercially available and low-cost materials. This objective is solved by an endoscope in accordance with claim 1 and by a visualization system in accordance with claim 13.

The disclosure relates to an endoscope comprising: a handle or interface, an insertion cord extending distally from the handle or interface, and a fluid pathway system comprising: a first flow-through part having a fluid conduit formed therein and comprising a receiver portion, a second flow-through part having a fluid conduit formed therein and comprising a tubular connector portion, and a retainer having an insertion end and a reception end, wherein the insertion end of the retainer is inserted in the receiver portion of the first flow-through part, wherein the tubular connector portion of the second flow-through part is inserted in the reception end of the retainer, and wherein the retainer is sized and configured to fluidly sealingly secure the second flow-through part to the first flow-through part.

The endoscope according to the disclosure is beneficial in that it enables high and reliable flow performance by superior sealing and low pressure loss. As an example, the fluid pathway system may be used to provide suction at the endoscope distal tip by connecting to a vacuum source. Medical vacuum at hospitals is often provided at a pressure of approximately 50 kPa below atmospheric pressure. For best performance of the endoscope pressure loss in the fluid pathway system should be kept low. High suction capability of the endoscope may be used to have a clear sight by the observation optical system by removing blood or mucus.

The endoscope according to the disclosure has the advantage that the fluid pathway system is fast and easy to assemble. Further the assembly does not require any special skills or education, as the assembly only requires that parts are pushed into engagement. The construction is also tolerant in that if parts are connected wrongly, they can be disassembled and reconnected, which lowers the scrap rate and hence lowers cost and environmental impact. An extra benefit is that the endoscope can be disassembled after use, which makes recycling of materials much more efficient and lowers the environmental impact. Further the parts are not subject to high forces, which could over time otherwise lead to aging and increase a potential risk of disconnection or leaks.

The receiver portion of the first flow-through part may be generally cylindrical with a receiver inner diameter, the tubular connector portion of the second flow-through part may have a connector outer diameter, the retainer may have a generally cylindrical outer surface and an inner space having a generally cylindrical inner surface, wherein the retainer may have an inner retainer diameter of the inner space and an outer retainer diameter of the outer surface, the retainer inner diameter may fit the connector portion outer diameter and the retainer outer diameter may fit the receiver portion inner diameter, so that the retainer may be configured to seal and secure the second flow-through part to the first flow-through part.

A flow-through part is any part provided with a fluid channel, such as a tube, a valve, a pipe connection, a pipe stub or a nozzle. In this context a "first flow-through part" is a part configured to receive a retainer and the "second flow-through part" is a part configured to be inserted into the retainer, i.e. the first flow-through part comprises a female connector, whereas the second flow-through part comprises a male connector. In some cases, the same part can constitute a "first flow-through part" as well as a "second flow-through part", if the part is configured to provide connection to two other flow-through parts by having both a female connector and a male connector.

In one example of the present disclosure, the retainer comprises smooth inner and outer surfaces. In another example of the present disclosure, however, the retainer comprises annular sealing protrusions on the inner surface and/or the outer surface. Annular sealing protrusions may provide for low friction insertion of the retainer and low friction uptake of parts in the retainer interior compared to a retainer having a smooth surface. Further annular sealing protrusions may add to the fluid tightness of the fluid pathway system. The retainer may have for example four annular protrusions, but in some cases it may suffice to have only one, two or three annular protrusions, whereas in other cases more than four annular protrusions may be provided, such as five to seven annular protrusions.

In one example of the present disclosure, the first flow-through part is a Y-connector, and the second flow-through part is a suction tube.

In one example of the present disclosure, the first flow-through part is a suction valve, and the second flow-through part is a suction tube and/or a vacuum hose fitting.

The endoscope may comprise an adhesive in the fluid pathway system, e.g. if considered expedient to avoid dislocation of the parts making up the fluid pathway system. It is preferred, however, that the fluid pathway system is essentially adhesive-free. That the fluid pathway system is essentially adhesive-free eliminates the risk of errors in production, such as adhesive flowing to locations, where it could negatively impact fluid flow. An adhesive-free system also has the benefit that it greatly increases the possible choice of materials, such as the use of silicone materials, which are inherently difficult to adhere to, and of course it also facilitates recycling by easy disassembly of parts.

The endoscope incorporates a retainer made of any suitable material, but according to an embodiment the retainer is made of a material having a hardness in the interval of 10-100 Shore A, preferably 50-70 Shore A, which is considered to provide a suitable compromise between ease of assembly, sealing capability and grip. A very low hardness can make it difficult to produce and handle the retainer. The hardness is measured according to ISO 868:2003.

The retainer comprises a flange at the reception end thereof. The flange aids during insertion of AM3227P-EP-0096 WG/-/FM : Ambu A/S [BS:-001-] the retainer and further indicates full and correct insertion of the retainer as the flange abuts the end of the receiver portion of the first flow-through part.

According to an embodiment the retainer comprises a lead-in zone to the inner space at the reception end. The lead-in zone may facilitate insertion of the second flow-through part in the retainer, e.g. by providing a funnel-shaped entry to the retainer inner space.

According to an embodiment, the retainer comprises a constriction at the insertion end thereof. The constriction may provide a seal between the first and second flow-through parts.

In one example, the inner interface between first flow-through part, second flow-through part and retainer constriction is configured to provide a consistent inner diameter.

To provide high sealing efficiency and grip, without assembly being too laborious, it is preferred that the retainer has a ratio of length to outer diameter in the range of 6:5 to 7:5. This interval is considered to provide good results. A ratio above 7:5 is possible and may improve sealing efficiency and grip of the retainer, but may make it more difficult to insert the retainer, e.g. because of longer distance to cover during insertion and potentially a risk of buckling of the retainer during insertion. A ratio below 6:5 is also possible, and may make insertion of the retainer easier, and potential disassembly after use easier, whereas potential lower sealing efficiency and grip of the retainer can be counteracted e.g. by adding further annular sealing protrusions, providing a tighter fit or using softer materials.

The retainer may be assembled of a plurality of parts, e.g. a separate shell part of a rigid material and separate flexible seal rings mounted thereon, however it is preferred that the retainer is molded in one piece from an elastic material, such as Liquid Silicone Rubber (LSR), preferably medical grade silicone. This is considered to be advantageous for cost of production and assembly, and medical grade silicone is considered safe to use in endoscopes. An example of a suitable material is LSR of the Elastosil^{®} LR 3003 series. Alternatively, all thermoplastic elastomers may be used, which may have some advantages in terms of sustainability, as such materials can be easily recycled, and the retainer may with this material be produced by injection molded.

The present disclosure also relates to a visualization system comprising an endoscope as described above, and a monitor connectable to the endoscope.

### BRIEF DESCRIPTION OF THE DRAWINGS

The disclosure will now be made in greater detail based on non-limiting exemplary embodiments and with reference to the schematic drawings on which:
Fig. 1 shows a side view of an endoscope connectable to a monitor to form a visualization system,
Fig. 2 shows the interior of a prior art endoscope handle,
Fig. 3 shows an endoscope according to the disclosure and the interior of the handle thereof,
Fig. 4 shows a fluid pathway system according to the disclosure,
Fig. 5 is a perspective view of a retainer according to the disclosure,
Fig. 6 is a longitudinal section of the retainer of Fig. 5, and
Fig. 7 is a longitudinal section of a detail of an assembled fluid pathway system corresponding to Fig. 4 and illustrating the retainer of Fig. 5 as fitted in the fluid pathway.

The figures are schematic in nature and serve only to understand the disclosure. Identical elements are marked with the same reference signs.

### DETAILED DESCRIPTION

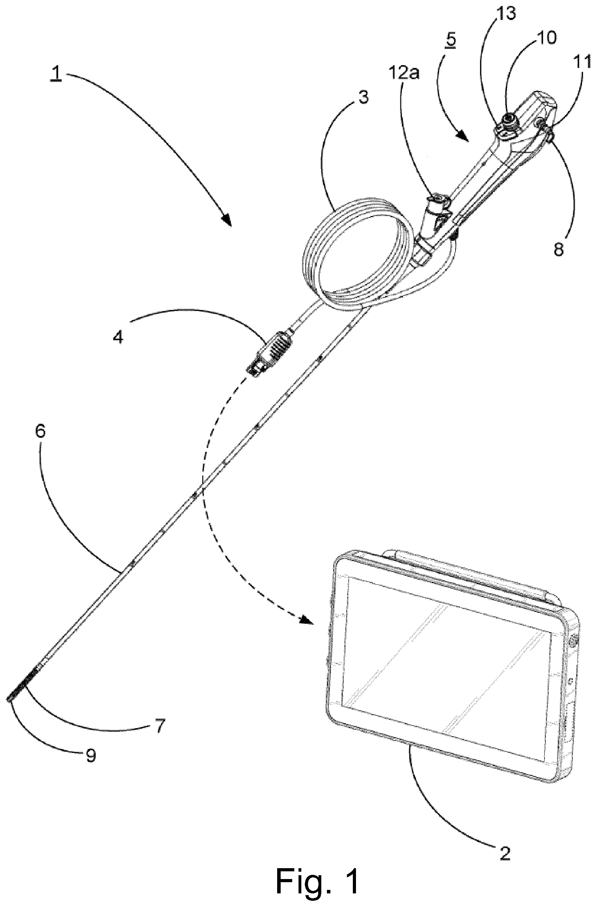

Fig. 1 illustrates a system comprising an endoscope 1 and a display unit 2. The endoscope 1 comprises an umbilical cord 3 and an electrical connector 4 for connecting to the display unit 2. The endoscope 1 is preferably single-use i.e. intended to be thrown away after use on one single patient, whereas the display unit may 2 be used multiple times with different single-use endoscopes. The endoscope 1 further comprises a handle 5 at a proximal end of the endoscope and an insertion cord 6 extending distally of the handle 5. At the distal end of the insertion cord 6 a bending section 7 is provided. The bending section 7 may be controlled by a controller 8 to deflect the distal end of the insertion cord 6. At the distal end of the bending section 7 a tip part 9 with optical system is provided. The optical system generally comprises a camera and illumination, such as LED's. As an alternative to arranging light sources, such as LED's, in the tip part, optical fibres may be provided for transporting light from a light source at the handle or outside the endoscope through the endoscope to the distal end thereof for illuminating an area of interest in front of the endoscope distal tip part 9. The insertion cord 6 may comprise one or more channels, e.g. a working channel for advancing tools through the endoscope and out through an opening at the distal end of the endoscope, and a fluid pathway, such as a suction channel. The suction channel may be used for transporting fluids away from a position at the distal end of the endoscope, through an opening at the distal end of the endoscope. The same channel may be used as working channel and suction channel. The handle 5 often also comprises a suction valve button 10 comprising an external valve cap forming a push-button for activating suction through the suction channel to an external vacuum source (not shown) via a vacuum hose connector 11. The handle 5 may as shown also comprise a biopsy port 12 with a biopsy cap 12a for insertion of an external tool through the handle and the working channel to exit at the opening at the distal end of the endoscope. Further the handle 5 may be provided with buttons for activation of electrical switches 13 controlling function of the image capture, such as taking of still images.

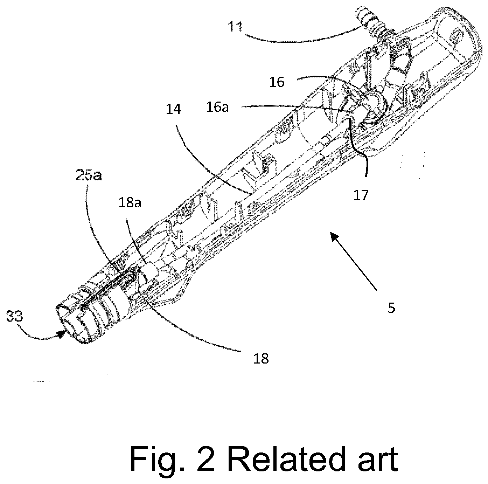

The interior of the handle 5 of a related art endoscope is illustrated in Fig. 2, showing a suction tube 14 connected at one end to a tube connector 16a of a valve 16 and at the other end to a tube connector 18a of a Y-connector 18. The suction tube 14 is connected to the respective parts by means of an adhesive, which is applied to the gaps between the assembled parts, e.g. a gap 17 between the tube 14 and the tube connector 16a. The adhesive secures and seals the connection of the tube to the tube connector.

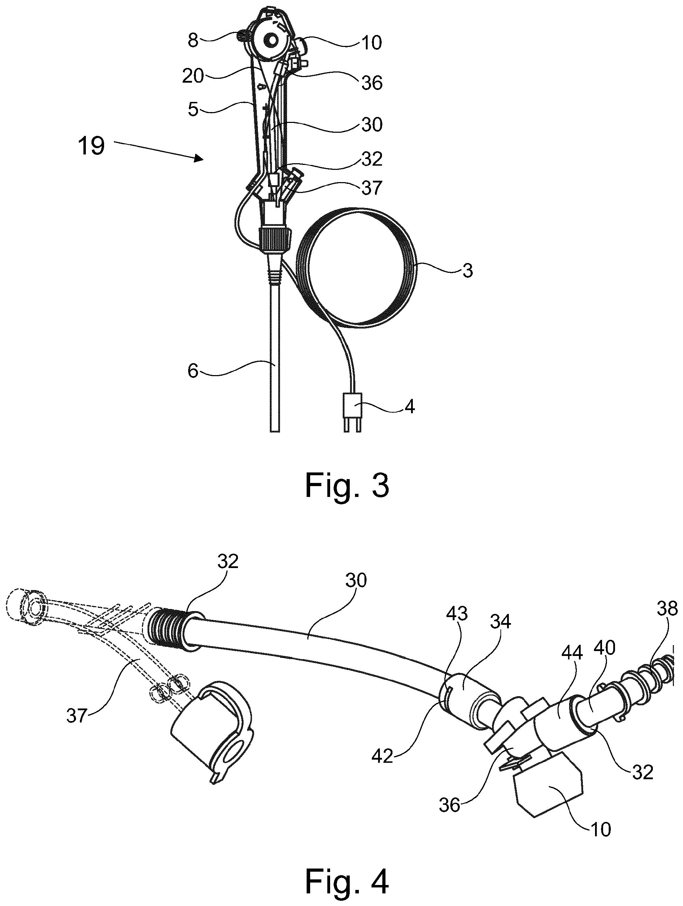

An endoscope 19 according to the present disclosure is illustrated in Fig. 3 and is generally similar to the endoscope 1 of Fig. 2. The endoscope 19 comprises umbilical cord 3 and connector 4, which is connectable to a display unit (not shown). The endoscope 19 comprises a handle 5 at a proximal end of the endoscope and an insertion cord 6 (schematically illustrated) extending distally of the handle 5. For illustration purposes part of the handle is removed to reveal the handle interior. A bending section of the insertion cord may be controlled by a controller 8 to deflect the distal end of the insertion cord 6 via steering wires 20. A fluid pathway system according to the present disclosure is arranged in the interior of the handle 5. The fluid pathway system according to the present disclosure is discussed in more detail in Fig. 4 illustrating the fluid pathway system in isolation.

The illustrated fluid pathway system comprises a valve 36, constituting an example of a first flow-through part, and a coupler of a Y-connector 37, constituting another example of a first flow-through part. The valve 36 comprises a tube coupler 34 at one end and a connector coupler 44 at the opposite end, both couplers configured to receive retainers 32. The valve 36 has a suction valve button 10 for activation of suction. A vacuum hose fitting 38, constituting an example of a second flow-through part, has a connector part 40, which is inserted in the retainer 32, which again is inserted into the connector coupler 44 to form a fluidly sealed connection therewith. In turn, a suction tube 30, constituting another example of a second flow-through part, is inserted in the second retainer 32 to form a fluidly sealed connection with the tube coupler 34.

The first flow-through part may be provided with a retention recess and the retainer may be provided with a retention protrusion matching the retention recess to further secure anchoring of the retainer in the coupler. A retention recess 42 is shown, receiving a retention protrusion 43 of the retainer 32. The retention recess may be a through-hole or an indentation on an inner surface of the first flow-through part, for example. The through-hole may be an elongate hole, such as a slot. More than one retention recess and matching retention protrusion may be provided. From an assembly standpoint, the retainer may be inserted first to facilitate placement of the retention protrusion into the retention recess. Subsequently the second flow-through part is inserted into the retainer, preferably overlapping the retention protrusion to ensure it remains in place under pressure or tension. However, the retainer may be slid over the second flow-through part first and the combination may then be inserted into the first flow-through part, so long as the first flow-through part is not so rigid as to prevent insertion of a retainer with a retention protrusion.

The other end of the suction tube 30 is inserted in a retainer 32, which is inserted in a coupler of a Y-connector 37, which constitutes another example of a second flow-through part.

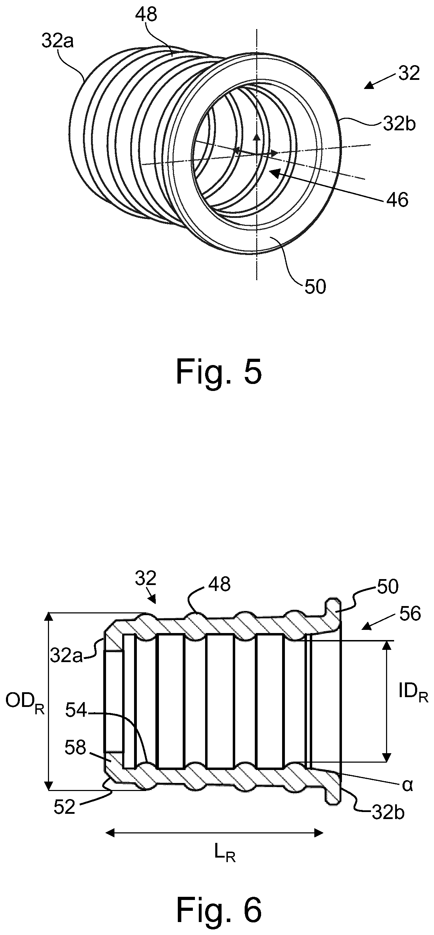

The retainer 32 will now be described in more detail with reference to Fig. 5 and 6. The retainer 32 has a generally cylindrical outer shape and a generally cylindrical inner space 46. By generally cylindrical outer shape is meant that the retainer 32 in a relaxed state will have essentially the same outer diameter along the length of the retainer. However, protrusions may be provided on the outer surface, such as annular ribs 48, and further a flange 50 is provided at the reception end 32b as illustrated. The annular ribs 48 are semi-circular, which is preferred at present, but it is considered that the protrusions may have any other shape, such as triangular or disk shaped. The retainer may further have a taper 52 at the insertion end 32a to facilitate insertion in the coupler. By generally cylindrical inner space should be understood that the retainer 32 in a relaxed state thereof will have essentially the same inner diameter along the length of the retainer. However, protrusions may be provided on the inner surface, such as annular ribs 54. Further, the entrance 56 to the inner space 46 may be slightly conical in shape as illustrated by angle α to facilitate insertion of the connector in the inner space. Additionally, the entry end 32a of retainer 32 may have a constriction 58 as shown, but is generally open with a through hole for the fluid flow.

As an example, the retainer may have a total length of 10-15 mm, such as 12.85 mm. The retainer may have an outer diameter, in including any potential protrusions, of 9-11 mm, such as 9.55 mm. The retainer may have an inner diameter, taking any potential protrusions into account, of 5-8 mm, such as 6.65 mm. Wall thickness of the retainer between any potential protrusions may be 0.75 mm to 1 mm, such as 0.89 mm. Wall thickness at potential protrusions may be 1.5 to 2.5 times the wall thickness between protrusions, such as approximately 1.8 times the wall thickness between protrusions. Through hole at the entry end 32a of the retainer 32 may have a diameter of 5-6 mm, such as 5.5 mm. Outer diameter of the flange (if any), may be 10-12 mm, such as 11.23 mm.

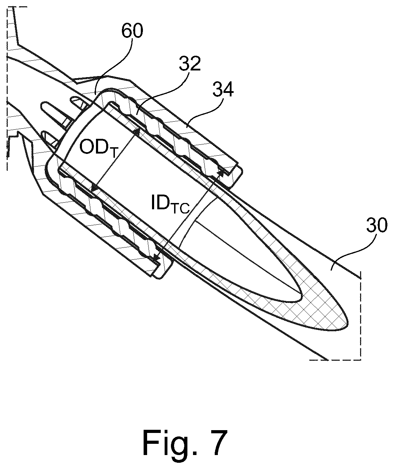

Fig. 7 illustrates a detail of an assembled fluid pathway system, namely a longitudinal section of the suction tube 30 fitted in the retainer 32, which in turn is fitted in the coupler 34 of valve 36 (see Fig. 3). The retainer 32 is configured to fit the interior of the coupler 34, and likewise the retainer 32 is configured to fit the exterior of the suction tube 30. In this embodiment the length of the retainer 32 corresponds to the length of the interior of the coupler 34, so the end of the retainer 32 abuts a step 60 of the coupler 34. The constriction 58 may act as a seal between the end of the suction tube 30 and the step 60 of the coupler 34. The coupler 34 is generally shell shaped, but it is conceivable to provide barbs or cut-outs in the inner surface of the coupler 34 for increased grip between the coupler 34 and the retainer 32, such as the cut-out 42 discussed above. As can be seen the fluid flow path through the assembled fluid pathway system is virtually unrestricted and smooth with the retainer constriction having an inner opening diameter configured to correspond to the inner diameter of other parts making up the fluid pathway system, such as the inner diameter of the suction tube, so the pressure loss is kept at a minimum.

Single-use endoscopes optimize workflow and reduce cost while saving patient's lives and improving patient care. They optimize workflow and reduce cost because they are always ready when needed without the traditional large-scale capital and repair budgets required for reusable endoscopes. For example, a sterilization and storage facility is avoided, there is no need to maintain evidence of sterilization, and there is no need to transport endoscopes from sterilization and storage facilities to the buildings where they are needed, sometimes in the middle of the night or weekends. They save patient's lives and improve patient care because they are readily available and do not pose a cross-contamination risk. This also reduces hospital re-admissions. While single-use endoscopes are disposed after a single patient use (one or more procedures may be performed while the patient remains in the treatment room), the environmental impact of re-useable endoscopes, due to cleaning materials, CO₂ emissions during the cleaning process, and use of disposable personal protective equipment by personnel involved in transportation and sterilization of the re-useable endoscopes, is similar to that of single-use endoscopes. Studies are emerging showing that the environmental impact of single-use endoscopes may, in fact, be less than that of re-usable endoscopes. To further reduce environmental impact, the endoscopes according to the present disclosure are primarily made of polymer materials.

### List of reference signs:

1 Endoscope
2 display unit
3 umbilical cord
4 electrical connector
5 handle
6 insertion cord
7 bending section
8 controller
9 tip part
10 suction valve button
11 vacuum hose connector
12 biopsy connector
12a biopsy cap
13 electrical switch
14 suction tube
16 suction valve
16a tube connector
17 gap
18 Y-connector
18a tube connector
30 suction tube
32 retainer
32a insertion end
32b reception end
34 tube coupler
36 valve
37 Y-connector
38 vacuum hose fitting
40 connector part
42 cut-out
43 retention protrusion
44 coupler
46 inner space
48 annular ribs
50 flange
52 taper
54 annular ribs
56 entrance
58 constriction
60 step
ID_{R} Inner diameter, retainer
OD_{R} Outer diameter, retainer
L_{R} Length, retainer
ID_{TC} Inner diameter, tube coupler
OD_{T} Outer diameter, tube
α angle

## Claims

1. An endoscope (1) comprising:
a handle (5) or interface,
an insertion cord (6) extending distally from the handle (5) or interface, and
a fluid pathway system comprising:
a first flow-through part (36, 37) having a fluid conduit formed therein and comprising a receiver portion (34, 44),
a second flow-through part (30, 38) having a fluid conduit formed therein and comprising a tubular connector portion,
a retainer (32) having an insertion end (32a) and a reception end (32b),
wherein the insertion end (32a) of the retainer (32) is inserted in the receiver portion (34, 44) of the first flow-through part (36, 37),
wherein the tubular connector portion of the second flow-through part (30, 38) is inserted in the reception end (32b) of the retainer (32),
wherein the retainer (32) is sized and configured to fluidly sealingly secure the second flow-through part (30, 38) to the first flow-through part (36, 37),
and wherein the retainer (32) comprises a flange (50) at the reception end (32b) thereof,
**characterized in that**
the flange (50) abuts the end of the receiver portion (34, 44) of the first flow-through part (36, 37).

2. The endoscope (1) according to claim 1, wherein the retainer (32) comprises annular sealing protrusions (48, 54) on the inner surface and/or the outer surface.

3. The endoscope (1) according to any one of the claims above, wherein the first flow-through part (36, 37) is a Y-connector (37), and the second flow-through part (30, 38) is a suction tube (30).

4. The endoscope (1) according to any one of the claims above, wherein the first flow-through part (36, 37) is a suction valve (36), and the second flow-through part (30, 38) is a suction tube (30) and/or a vacuum hose fitting (38).

5. The endoscope (1) according to any one of the claims above, wherein the fluid pathway system is adhesive-free.

6. The endoscope (1) according to any one of the claims above, wherein the retainer (32) is made of a material having a hardness in the interval of 10-100 Shore A, preferably 50-70 Shore A.

7. The endoscope (1) according to any one of the claims above, wherein the retainer (32) comprises a lead-in zone providing a funnel-shaped entry to a retainer inner space at the reception end (32b).

8. The endoscope (1) according to any one of the claims above, wherein the retainer (32) comprises a constriction (58) at the insertion end (32a) thereof.

9. The endoscope (1) according to claim 8, wherein an inner interface between first flow-through part (36, 37), second flow-through part (30, 38) and retainer constriction (58) is configured to provide a consistent inner diameter.

10. The endoscope (1) according to any one of the claims above, wherein the retainer (32) has a ratio of length (L_{R}) to outer diameter (OD_{R}) in the range of 6:5 to 7:5.

11. The endoscope (1) according to any one of the claims above, wherein the retainer (32) is molded in one piece from an elastic material, such as medical grade silicone.

12. The endoscope (1) according to any one of the claims above, wherein the receiver portion (34, 44) has a generally cylindrical shape and a receiver inner diameter (ID_{TC}), wherein the tubular connector portion has a connector outer diameter (OD_{T}), wherein the retainer (32) has a generally cylindrical outer surface and an inner space (46) having a generally cylindrical inner surface, wherein the retainer (32) has an inner retainer diameter (ID_{R}) of the inner space (46) and an outer retainer diameter (OD_{R}) of the outer surface, and wherein the retainer inner diameter (ID_{R}) fits the connector portion outer diameter (OD_{T}) and the retainer outer diameter (OD_{R}) fits the receiver inner diameter (ID_{TC}) to form a fluid seal between the second flow-through part (30, 38), the retainer (32) and the first flow-through part (36, 37).

13. A visualization system comprising:
an endoscope (1) according to claim 1; and
a monitor (2) connectable to the endoscope (1).

## Patentansprüche

1. Endoskop (1) mit:
einem Griff (5) oder einer Schnittstelle,
einem Einführstrang (6), der sich vom Griff (5) oder der Schnittstelle distal erstreckt, und
einem Fluidleitungssystem, das umfasst:
ein erstes Durchflussteil (36, 37) mit einem darin ausgebildeten Fluidkanal, das einen Aufnahmeabschnitt (34, 44) umfasst,
ein zweites Durchflussteil (30, 38) mit einem darin ausgebildeten Fluidkanal, das einen rohrförmigen Verbinderabschnitt umfasst,
eine Halterung (32) mit einem Einführende (32a) und einem Aufnahmeende (32b),
wobei das Einführende (32a) der Halterung (32) in den Aufnahmeabschnitt (34, 44) des ersten Durchflussteils (36, 37) eingeführt ist,
wobei der rohrförmige Verbinderabschnitt des zweiten Durchflussteils (30, 38) in das Aufnahmeende (32b) der Halterung (32) eingeführt ist,
wobei die Halterung (32) so dimensioniert und ausgebildet ist, dass sie das zweite Durchflussteil (30, 38) fluiddicht am ersten Durchflussteil (36, 37) befestigt,
und wobei
die Halterung (32) an ihrem Aufnahmeende (32b) einen Flansch (50) aufweist,
**dadurch gekennzeichnet, dass**
der Flansch (50) an das Ende des Aufnahmeabschnitts (34, 44) des ersten Durchflussteils (36, 37) anstößt.

2. Endoskop (1) nach Anspruch 1, wobei die Halterung (32) ringförmige Dichtungsvorsprünge (48, 54) an der Innenfläche und/oder der Außenfläche aufweist.

3. Endoskop (1) nach einem der vorstehenden Ansprüche, wobei das erste Durchflussteil (36, 37) ein Y-Verbinder (37) ist und das zweite Durchflussteil (30, 38) ein Absaugschlauch (30) ist.

4. Endoskop (1) nach einem der vorstehenden Ansprüche, wobei das erste Durchflussteil (36, 37) ein Saugventil (36) ist und das zweite Durchflussteil (30, 38) ein Absaugschlauch (30) und/oder eine Vakuumschlauchanschlussstück (38) ist.

5. Endoskop (1) nach einem der vorstehenden Ansprüche, wobei das Fluidleitungssystem klebstofffrei ist.

6. Endoskop (1) nach einem der vorstehenden Ansprüche, wobei die Halterung (32) aus einem Material besteht, dessen Härte im Bereich von 10 bis 100 Shore A, vorzugsweise von 50 bis 70 Shore A, liegt.

7. Endoskop (1) nach einem der vorstehenden Ansprüche, wobei die Halterung (32) einen Einführbereich aufweist, der am Aufnahmeende (32b) einen trichterförmigen Zugang zu einem Innenraum der Halterung bildet.

8. Endoskop (1) nach einem der vorstehenden Ansprüche, wobei die Halterung (32) an ihrem Einführende (32a) eine Verengung (58) aufweist.

9. Endoskop (1) nach Anspruch 8, wobei eine innere Schnittstelle zwischen dem ersten Durchflussteil (36, 37), dem zweiten Durchflussteil (30, 38) und der Verengung (58) der Halterung so ausgebildet ist, dass sie einen gleichbleibenden Innendurchmesser bildet.

10. Endoskop (1) nach einem der vorstehenden Ansprüche, wobei die Halterung (32) ein Verhältnis von Länge (L_{R}) zu Außendurchmesser (OD_{R}) im Bereich von 6:5 bis 7:5 aufweist.

11. Endoskop (1) nach einem der vorstehenden Ansprüche, wobei die Halterung (32) einteilig aus einem elastischen Material, wie beispielsweise medizinischem Silikon, geformt ist.

12. Endoskop (1) nach einem der vorstehenden Ansprüche, wobei der Aufnahmeabschnitt (34, 44) eine im Allgemeinen zylindrische Form und einen inneren Aufnahmedurchmesser (ID_{TC}) aufweist, wobei der rohrförmige Verbinderabschnitt einen äußeren Durchmesser (OD_{T}) des Verbinders aufweist, wobei die Halterung (32) eine im Wesentlichen zylindrische Außenfläche und einen Innenraum (46) mit einer im Wesentlichen zylindrischen Innenfläche aufweist, wobei die Halterung (32) einen inneren Halterungsdurchmesser (ID_{R}) des Innenraums (46) und einen äußeren Halterungsdurchmesser (OD_{R}) der Außenfläche aufweist, und wobei der Innendurchmesser (ID_{R}) der Halterung auf den Außendurchmesser des Verbinderabschnitts (OD_{T}) passt und der Außendurchmesser der Halterung (OD_{R}) auf den Innendurchmesser des Aufnahmeteils (ID_{TC}) passt, um eine Fluiddichtung zwischen dem zweiten Durchflussteil (30, 38), der Halterung (32) und dem ersten Durchflussteil (36, 37) zu bilden.

13. Ein Visualisierungssystem, umfassend:
ein Endoskop (1) nach Anspruch 1; und
einen Monitor (2), der mit dem Endoskop (1) verbindbar ist.

## Revendications

1. Endoscope (1) comprenant :
une poignée (5) ou une interface,
un cordon d'insertion (6) s'étendant de manière distale à partir de la poignée (5) ou de l'interface, et
un système de circulation de fluides comprenant :
une première partie d'écoulement (36, 37) présentant un conduit de fluide formé à l'intérieur et comprenant une partie réceptrice (34, 44),
une deuxième partie d'écoulement (30, 38) présentant un conduit de fluide formé à l'intérieur et comprenant une partie de connecteur tubulaire,
un dispositif de retenue (32) présentant une extrémité d'insertion (32a) et une extrémité de réception (32b),
dans lequel l'extrémité d'insertion (32a) du dispositif de retenue (32) est insérée dans la partie réceptrice (34, 44) de la première partie d'écoulement (36, 37),
dans lequel la partie de connecteur tubulaire de la deuxième partie d'écoulement (30, 38) est insérée dans l'extrémité de réception (32b) du dispositif de retenue (32),
dans lequel le dispositif de retenue (32) est dimensionné et configuré pour fixer de manière étanche la deuxième partie d'écoulement (30, 38) à la première partie d'écoulement (36, 37),
et dans lequel le dispositif de retenue (32) comprend une bride (50) à son extrémité de réception (32b),
**caractérisé en ce que**
la bride (50) bute contre l'extrémité de la partie réceptrice (34, 44) de la première partie d'écoulement (36, 37).

2. Endoscope (1) selon la revendication 1, dans lequel le dispositif de retenue (32) comprend des protubérances d'étanchéité annulaires (48, 54) sur sa surface interne et/ou sa surface externe.

3. Endoscope (1) selon l'une quelconque des revendications ci-dessus, dans lequel la première partie d'écoulement (36, 37) est un connecteur en Y (37), et la deuxième partie d'écoulement (30, 38) est un tube d'aspiration (30).

4. Endoscope (1) selon l'une quelconque des revendications ci-dessus, dans lequel la première partie d'écoulement (36, 37) est une valve d'aspiration (36), et la deuxième partie d'écoulement (30, 38) est un tube d'aspiration (30) et/ou un raccord de tuyau à vide (38).

5. Endoscope (1) selon l'une quelconque des revendications ci-dessus, dans lequel le système de passage de fluide est sans adhésif.

6. Endoscope (1) selon l'une quelconque des revendications ci-dessus, dans lequel le dispositif de retenue (32) est fait d'un matériau présentant une dureté comprise entre 10 et 100 Shore A, de préférence entre 50 et 70 Shore A.

7. Endoscope (1) selon l'une quelconque des revendications ci-dessus, dans lequel le dispositif de retenue (32) comprend une zone d'entrée fournissant une entrée en forme d'entonnoir vers un espace intérieur du dispositif de retenue à l'extrémité de réception (32b).

8. Endoscope (1) selon l'une quelconque des revendications ci-dessus, dans lequel le dispositif de retenue (32) comprend un rétrécissement (58) à l'extrémité d'insertion (32a) de celui-ci.

9. Endoscope (1) selon la revendication 8, dans lequel une interface interne entre la première partie d'écoulement (36, 37), la deuxième partie d'écoulement (30, 38) et le rétrécissement de retenue (58) est configurée pour fournir un diamètre interne constant.

10. Endoscope (1) selon l'une quelconque des revendications ci-dessus, dans lequel le dispositif de retenue (32) présente un rapport longueur (L_{/R})/diamètre extérieur (DE/_{R}) compris dans la plage de 6:5 à 7:5.

11. Endoscope (1) selon l'une quelconque des revendications ci-dessus, dans lequel le dispositif de retenue (32) est moulé en une seule pièce à partir d'un matériau élastique, tel que du silicone de qualité médicale.

12. Endoscope (1) selon l'une quelconque des revendications ci-dessus, dans lequel la partie réceptrice (34, 44) présente une forme généralement cylindrique et un diamètre intérieur de récepteur (ID_{TC}), dans lequel la partie de connecteur tubulaire présente un diamètre extérieur de connecteur (OD_{T}), dans lequel le dispositif de retenue (32) présente une surface extérieure généralement cylindrique et un espace intérieur (46) présentant une surface intérieure généralement cylindrique, dans lequel le dispositif de retenue (32) présente un diamètre intérieur de retenue (ID_{R}) de l'espace intérieur (46) et un diamètre extérieur de retenue (OD_{R}) de la surface extérieure, et dans lequel le diamètre intérieur de retenue (ID_{R}) correspond au diamètre extérieur de la partie de connecteur (OD_{T}) et le diamètre extérieur de retenue (OD_{R}) correspond au diamètre intérieur du récepteur (ID_{TC}) pour former un joint de fluide entre la deuxième partie d'écoulement (30, 38), le dispositif de retenue (32) et la première partie d'écoulement (36, 37).

13. Système de visualisation comprenant :
un endoscope (1) selon la revendication 1; et
un moniteur (2) pouvant être connecté à l'endoscope (1).
